# EUROPEAN PATENT SPECIFICATION

(11) **EP 2 808 342 B1**
(45) Date of publication and mention of the grant of the patent: **10.10.2018**
(21) Application number: 14169902.5
(22) Date of filing: 26.05.2014
(51) Int. Cl.: C12N 5/00, G01N 33/50

(54) **Medium for in-vitro toxicity testing and in-vitro toxicity test method using the same**
Medium für In-vitro-Toxizitätstests und In-vitro-Toxizitätstestverfahren damit
Support pour tests de toxicité in vitro et procédé de test de toxicité in vitro utilisant celui-ci

(30) Priority: 31.05.2013 KR 20130062640
(43) Date of publication of application: 03.12.2014
(73) Proprietor: Daegu Gyeongbuk Institute of Science and Technology, Daegu 711-873 (KR)
(72) Inventor: Jeon, Won Bae, 711-873 Daegu (KR); Choi, Seong Kyoon, 711-857 Daegu (KR)
(74) Representative: Michalski Hüttermann & Partner Patentanwälte mbB

(56) References cited:
- US-A- 5 670 483
- US-A1- 2007 099 840
- DATABASE Geneseq [Online] 16 February 2012 (2012-02-16), "Human fibronectin peptide #1.", XP002727448, retrieved from EBI accession no. GSP:AZQ39785 Database accession no. AZQ39785 & KR 2011 0130274 A (DAEGU GYEONGBUK INST SCIENCE [KR]) 5 December 2011 (2011-12-05)
- DATABASE Geneseq [Online] 19 December 2013 (2013-12-19), "Elastin-like polypeptide (ELP) #11.", XP002727449, retrieved from EBI accession no. GSP:BAR75970 Database accession no. BAR75970 & KR 2013 0047404 A (DAEGU GYEONGBUK INST SCIENCE [KR]) 8 May 2013 (2013-05-08)
- FRANCESCO PAMPALONI ET AL: "Three-Dimensional Tissue Models for Drug Discovery and Toxicology", RECENT PATENTS ON BIOTECHNOLOGY, vol. 3, no. 2, 1 June 2009 (2009-06-01), pages 103-117, XP055128689, ISSN: 1872-2083, DOI: 10.2174/187220809788700201
- THORSTEN BUHRKE ET AL: "In vitro toxicological characterization of perfluorinated carboxylic acids with different carbon chain lengths", TOXICOLOGY LETTERS, vol. 218, no. 2, 4 February 2013 (2013-02-04), pages 97-104, XP055128685, ISSN: 0378-4274, DOI: 10.1016/j.toxlet.2013.01.025

## Description

The present invention relates to the use of a medium for in-vitro toxicity testing, and an in-vitro toxicity test method using the medium. More particularly, the present invention relates to the use of a medium for in-vitro toxicity testing which is designed in consideration of cell-to-cell interaction so that it can provide test results nearer to those obtained in practical in-vivo toxicity tests than can conventional in-vitro toxicity tests.

With an increase in the likelihood that a potentially hazardous substance will be incorporated to foods and medicinal products, there is an urgent need for evaluating a given substance for safety. However, due to increasing global concern about animal welfare, animal experiments tend to be excluded from as many studies as possible, except for indispensable cases. Accordingly, there has been a significant rise of in-vitro models substitutable for animal experiments.

Particularly, toxicity assays of biological or chemical substances are the most severely restricted from being applied to animals, and thus there is an increasing interest in developing models for use in in-vitro toxicity tests.

In this regard, bioengineering fields taking advantage of culturing techniques of animal cells are divided largely into three classes. The first class involves culturing various animal cells including genetically recombinant cell lines on a mass scale to produce useful substances such as antibodies, vaccines, growth hormones, immunomodulators (cytokines), antibiotics, etc. Culturing processes of animal cells, although economically or technically more difficult than those of microbial cells, have an advantage in terms of evaluating pharmacological actions with regard to immunological properties or side effects.

Next, the second class involves tissue engineering in which animal cells or tissues themselves are utilized as biomass products, as in the implantation of bone marrow or development of artificial organs. These biomass products may be applied with a clinical or a non-clinical purpose. For the latter, they can be used in the study of histogenesis-related mechanisms or functions, pharmacological metabolism, or toxicological assays.

The third class is a combination of the two above-mentioned classes, as exemplified by gene therapy or cellular therapy in which cells or tissues, genetically engineered to have enhanced specific functions, are implanted into the body.

Conventional in-vitro toxicity tests are carried out with a toxic substance of interest applied to animal cells, which are cultured on polystyrene-coated plates. However, all of the cells grown in this environment are prone to intensive exposure to a toxic substance even when a small amount of the toxic substance is employed, so that there is a high possibility of exerting higher toxicity than is practical in-vivo.

KR 2011 0130274 A discloses a multiple block biopolymer. US 2007/099840 A1 discloses a hydrogel composition comprising an aqueous dispersion phase and a plurality of peptides, or derivatives, or analogues thereof, wherein each peptide comprises at least two amino acid residues and an aromatic stacking ligand, and wherein the hydrogel is formed by self-assembly of said peptides in said aqueous dispersion medium.

Accordingly, there is an urgent need for an in-vitro toxicity test method that guarantees toxicity results more similar to those that would be obtained in the human body.

Accordingly, the present invention has been made keeping in mind the above problems occurring in the prior art, and an object of the present invention is to provide an in-vitro toxicity test method which guarantees test results nearer those obtained from in-vivo toxicity testing than do conventional in-vitro toxicity methods.

Also, the present invention aims to provide a medium for in-vitro toxicity testing which can be used in the method.

The present invention provides an in-vitro toxicity test method, comprising: (1) coating a cell culture dish with an artificial elastin-like extracellular matrix, wherein the artificial elastic-like extracellular matrix comprises a protein having the amino acid sequence set forth in the following General Formula 1: [General Formula 1] TGPG[VGRGD(VGVPG)₆]₂₀; (2) culturing blast cells in the coated culture dish to a form a three-dimensional cell cluster; and (3) measuring a toxic substance for toxicity to the three-dimensional cell cluster wherein the toxic substance of step (3) comprises at least one selected from the group consisting of: PFOA (perfluorooctanoic acid) and PFOS (perfluorooctane sulfonate).

According to another preferred embodiment of the present invention, the cell culture dish may be made of at least one material selected from the group consisting of: polystyrene, stainless steel, glass and a biopolymer.

In another preferred embodiment of the present invention, the artificial elastin-like extracellular matrix is applied at an average thickness of 0.001 µm to 10 µm to the culture dish in step (1).

In another preferred embodiment of the present invention, the blast cells of step (2) may be selected from the group consisting of: neuroblasts, angioblasts, myoblasts, myeloblasts, fibroblasts, osteoblasts, chondroblasts, and a combination thereof.

In another preferred embodiment of the present invention, the neuroblasts may include at least one selected from the group consisting of: N2a, SH-SY5Y, U937, Sk-Mel, A549, BEAS-2, HepG2, beta-TC, HIT-T15, HEK 293, adult stem cells, and embryonic stem cells.

In another preferred embodiment of the present invention, the blast cells of step (2) may be cultured at 20°C to 45°C for 12 hrs to 240 hrs.

In another preferred embodiment of the present invention, the coated cell culture dish of step (2) may contain at least one selected from the group consisting of: Eagle's Minimum Essential Medium, glutamine, sodium pyruvate, sodium bicarbonate, penicillin, and streptomycin.

In another preferred embodiment of the present invention, the toxicity of step (3) is measured using at least one selected from the group consisting of: an inhibitory concentration (IC₅₀) assay, a cell apoptosis assay, an active oxygen assay, a cytokine assay, and a combination thereof, with preference for an inhibitory concentration (IC₅₀) assay.

Also, the present invention provides the use of a medium for an in-vitro toxicity test, the medium comprising: an artificial elastin-like extracellular matrix-coated culture dish for 3D cell clusters, wherein the artificial elastic-like extracellular matrix comprises a protein having the amino acid sequence set forth in the following General Formula 1: [General Formula 1] TGPG[VGRGD(VGVPG)₆]₂₀; and a 3D cell cluster grown in the culture dish.

In another preferred embodiment of the present invention, the 3D cell cluster comprises at least one selected from the group consisting of: neuroblasts, angioblasts, myoblasts, myeloblasts, fibroblasts, osteoblasts, chondroblasts, and a combination thereof.

In another preferred embodiment of the present invention, the artificial elastin-like extracellular matrix is applied at an average thickness of 0.001 µm to 10 µm to the culture dish.

Prior to the elucidation of the present invention, terms used herein are defined as follows.

As used herein, the term "extracellular matrix (ECM)" refers to an intercellular part having a network structure composed mainly of proteins and polysaccharides.

As used herein, the term "artificial extracellular matrix" refers to an extracellular matrix, which plays an important role in cell attachment, migration and differentiation, artificially prepared by means of, for example, gene cloning; and is intended to encompass an artificial protein containing the arginyl glycyl aspartic acid (Arg-Gly-Asp (RGD)) motif involved in integrin mediated interaction, with an elastin mimetic protein serving as a backbone.

The term "blast cell," as used herein, refers to a cell that is undifferentiated or differentiated only up to several times, and that is rich in RNA, with the onset of vigorous DNA synthesis. In addition, "blast cell" is intended to encompass a cell that synthesizes and secretes collagenous fibers, like a fibroblast.

In addition, the term "3-dimensional cell cluster" means an aggregation of cells with a three-dimensional pattern, which is artificially constructed to resemble a bio-tissue by inducing cell-to-cell interaction based on the expression of gap junction proteins, such as cadherin and connexin. Whereas a 2D cell cluster has a monolayer of cells, a 3D cell cluster has multilayered cells. Hence, 3D cell clusters are higher in cell density per unit area than are 2D cell clusters.

In conventional in-vitro toxicity tests, all cells of interest are intensively exposed to a toxic substance even when it is used in a small amount; and thus are difficult to apply to the inference of practical toxic effects in-vivo. Designed in consideration of cell-to-cell interaction, however, the in-vitro toxicity test of the present invention can provide test results nearer to those obtained in practical in-vivo toxicity tests than can conventional in-vitro toxicity tests.

Below, a detailed description will be given of the present invention, with reference to the accompanying drawing.

As mentioned above, conventional in-vitro toxicity tests are problematic in that all cells of interest are intensively exposed to a toxic substance even when it is used in a small amount, and thus are difficult to apply to the inference of practical, in-vivo toxic effects.

The present invention addresses an in-vitro toxicity test method, comprising: (1) coating a cell culture dish with an artificial elastin-like extracellular matrix; (2) culturing blast cells in the coated culture dish to a form a three-dimensional cell cluster; and (3) measuring a toxic substance for toxicity to the three-dimensional cell cluster; whereby test results can be obtained as more approximate values to the actual in-vivo toxicity than can those obtained by conventional in-vitro toxicity test methods.

The in-vitro toxicity test method of the present invention will be explained.

First, in step 1, a cell culture dish is coated with an artificial elastin-like extracellular matrix. The artificial elastin-like extracellular matrix is configured to enhance interaction between cells grown in the cell culture dish. The artificial elastin-like extracellular matrix contains an amino acid sequence as set forth in the following General Formula 1.

[General Formula 1] TGPG[VGRGD(VGVPG)₆]₂₀.

The amino acid sequence used in the present invention is expressed with abbreviations for amino acids according to the IUPAC-IUB nomenclature.

| IUPAC-IUB Name | One-Letter Notation | IUPAC-IUB Name | One-Letter Notation | IUPAC-IUB Name | One-Letter Notation |
|---|---|---|---|---|---|
| Alanine | A | Glycine | G | Proline | P |
| Arginine | R | Histidine | H | Serine | S |
| Asparagine | N | Isoleucine | I | Threonine | T |
| Aspartic acid | D | Leucine | L | Tryptophan | W |
| Cysteine | C | Lysine | K | Tyrosine | Y |
| Glutamic acid | E | Methionine | M | Valine | V |
| Glutamine | Q | phenylalanine | F | | |

In addition, the cell culture dish may be made of any material that is typically used in the art. Preferable choices may be at least one selected from the group consisting of:, but not limited to, polystyrene, stainless steel, glass, and a biopolymer. Examples of the biopolymer include cellulose, chitin, heparin, and silk.

No particular limitations are imposed on the shape of the cell culture dish so long as it is typically available for cell culture. Preferably, the cell culture dish may be a shell (schale), a test tube, a cell culture flask or a cell culture plate, but is not limited thereto.

Also, the cell culture dish may have any size so long as it allows the cells to adequately grow. Preferably, the size of the cell culture dish may be on the order of 0.32 to 20 cm², but is not limited thereto.

Furthermore, no particular limitations are imposed on the amount of the artificial elastin-like extracellular matrix applied to the cell culture dish. Preferably, the cell culture dish may be coated with 10 µl/well to 100 µl/well of the artificial elastin-like extracellular matrix at a concentration of 0.5 µM to 10 µM.

The solvent available for coating the cell culture dish with the artificial elastin-like extracellular matrix is not particularly limited if it is typically used. Phosphate buffered saline (PBS) or water may be used, with preference for PBS.

So long as it is acceptable for cell culture dishes, any coating thickness of the artificial elastin-like extracellular matrix may be employed. It may preferably range from 0.001 µm to 10 µm.

Turning to step 2, it is adapted to culture blast cells in the coated cell culture dish to form a 3D cell cluster. The blast cells are not specifically limited if they are sensitive to a toxic substance and can grow to a cell cluster. For toxicity tests according to organs, cells originating from the corresponding organs may be used. Preferably, the blast cells may include at least one selected from the group consisting of: neuroblasts, angioblasts, myoblasts, myeloblasts, fibroblasts, osteoblasts, and chondroblasts, with more preference for neuroblasts.

When the blast cells are neutoblasts, they may include at least one selected from the group consisting of: N2a, SH-SY5Y, U937, Sk-Mel, A549, BEAS-2, HepG2, beta-TC, HIT-T15, HEK 293, adult stem cells and embryonic stem cells, with preference for N2a cells.

In addition, any density at which the blast cells are seeded to the cell culture dish is not particularly limited if it is acceptable for typical use. Preferably, the density may be on the order of 1 × 10⁵ cells/well to 3 × 10⁴ cells/well and more preferably on the order of 1 × 10⁴ cells/well to 2.5 × 10⁴ cells/well.

With regard to culture conditions of the blast cells, they may include those typically used for cell culture, without limitations. Preferably, the blast cells may be cultured at 20°C to 45°C for 12 hrs to 240 hrs, and more preferably at 30°C to 40°C for 24 hrs to 120 hrs.

If the blast cells are cultured at less than 20°C, they may tend to be lysed in the cell culture medium of the artificial elastin-like extracellular matrix so that they are unlikely to form a 3D cell cluster. On the other hand, a temperature exceeding 45°C cannot guarantee a suitable environment for cell culture, inhibiting cell proliferation and 3D cell clustering.

When formed, the 3D cell cluster may preferably have an average diameter of 20 µm to 400 µm, and more preferably in the order of 80 µm to 300 µm.

Finally, step 3 is intended to add a toxic substance to the 3D cell cluster to measure the toxic effect of the substance on the 3D cell cluster selected from the group consisting of: perfluorooctanoic acid (PFOA) and perfluorooctane sulfonate (PFOS).

In addition, the amount of the toxic substance added to the 3D cell cluster is not particularly limited if it is usually used for toxicity tests. Preferably, its amount may range from 10 µM to 1500 µM.

No particular limitations are imposed on the method of measuring a substance for toxicity so long as it is conventionally used. Preferably, it may be selected from the group consisting of: an inhibitory concentration (IC₅₀) assay, a cell apoptosis assay, an active oxygen assay, a cytokine assay, and a combination thereof, with preference for an inhibitory concentration (IC₅₀) assay.

The medium for in-vitro toxicity tests useful for the method may comprise an artificial elastin-like extracellular matrix-coated culture dish for 3D cell clusters; and a 3D cell cluster grown in the culture dish.

The artificial elastin-like extracellular matrix is configured to enhance interaction between cells grown in the cell culture dish. The artificial elastin-like extracellular matrix contains an amino acid sequence as set forth in the following General Formula 1.

[General Formula 1] TGPG[VGRGD(VGVPG)₆]₂₀.

Furthermore, no particular limitations are imposed on the amount of the artificial elastin-like extracellular matrix contained in the medium. Preferably, the artificial elastin-like extracellular matrix has a concentration of 0.5 µM to 10 µM. More preferably, the cell culture dish may contain 10 µl to 100 µl of an artificial elastin-like extracellular matrix with a concentration of 0.5 µM to 10 µM.

In addition, if the cell culture dish is as conventionally used, the material of which the cell culture dish is made is not particularly limited.

The 3D cell cluster may preferably have an average diameter of 20 µm to 400 µm, and more preferably 80 µm to 300 µm.

If it is an aggregation of cells that are able to detect toxicity, any 3D cell cluster may be used without particular limitations. It may be an aggregation of cells selected from the group consisting of: neuroblasts, angioblasts, myoblasts, myeloblasts, fibroblasts, osteoblasts, and chondroblasts; with neuroblasts being preferred.

Examples of the blast cells include N2a, SH-SY5Y, U937, Sk-Mel, A549, BEAS-2, HepG2, beta-TC, HIT-T15, HEK 293, adult stem cells, and embryonic stem cells, with preference for N2a.

Designed to provide cell-to-cell interaction, the 3D cell cluster of the present invention has significant advantage over 2D cell aggregates in that when applied to an in-vitro toxicity test, it guarantees a toxicity result nearer to a practical in-vivo value (Test Examples 1 and 2) .

A natural extracellular matrix is composed of collagen type I, IV, fibronectin, and laminin. When the cell culture dish is coated with a matrix other than the artificial elastin-like extracellular matrix of the present invention, no 3D cell clusters may be formed. A 3D cell cluster, even if formed with a matrix other than the artificial elastin-like extracellular matrix, is quantitatively too small to perform an in-vitro toxicity test.

Further, the artificial elastin-like extracellular matrix of the present invention enables cells to be cultured in a suspension manner, but not in an adherent manner, because of the hydrophobicity of the elastin-like protein. In addition, the RGD motif of the artificial elastin-like extracellular matrix binds to the integrin receptor of cells to stimulate cell signaling pathways, increasing cell activity and cell-to-cell interaction and thus encouraging the formation of 3D cell clusters.

A better understanding of the present invention may be obtained through the following examples which are set forth to illustrate, but are not to be construed as limiting the present invention.

### EXAMPLE 1: Synthesis of Artificial Elastin-like Extracellular Matrix and Coating of Cell Culture Dish therewith

For use as an artificial elastin-like extracellular matrix, TGPG[VGRGD(VGVPG)₆]₂₀ was synthesized using four oligonucleic acid salts. In order to construct a unit gene corresponding to the seven pentapeptides VGRGD(VGVPG)₆, the four oligonucleic acids were chemically synthesized, amplified, and linked to each other. That is, the four oligonucleic acid salts were manipulated to construct a unit gene encoding the seven pentapeptides VGRGD(VGVPG)₆. Then, the unit gene was consecutively repeated and manipulated into a gene encoding TGPG [VGRGD (VGVPG)₆]₂₀ in pUC19. The resulting gene was cloned into pET-25(+)-1 (Novagen, USA).

After transformation of the recombinant plasmid to E. coli BLR(DE3), the cell was cultured in 40 liters of TB (terrific broth) to express TGPG[VGRGD(VGVPG)₆]₂₀. This protein was isolated in PBS (phosphate-buffered saline, Gibco, USA) pH 7.2 by reverse-phase transition. The artificial elastin-like extracellular matrix TGPG[VGRGD(VGVPG)₆]₂₀ was designated REP.

REP was dissolved at a concentration of 10 µM in phosphate buffered saline (PBS), and the 10 µM REP solution was aliquoted in an amount of 50 µl per well into 96-well polystyrene plates and incubated for 1 hr at 37°C. The supernatant was removed from each well to leave REP to adhere to the plates.

### EXAMPLE 2: Culturing of 3D Cell Cluster

N2a cells (CCL-121, purchased from the ATCC, USA) were seeded at a density of 5x10⁵ cells/well into the culture plates coated with REP prepared in Example 1 (hereinafter referred to as "REP-plate"). The cell culture medium was Eagle's Minimum Essential Medium (EMEM) supplemented with 2 mM glutamine, 1 mM sodium pyruvate, 1.5 g/l sodium bicarbonate, 100 units/ml penicillin and 100 µg/ml streptomycin. The REP-plates where N2a cells were seeded were incubated for 3 days at 37°C in a 5% CO₂ atmosphere with humidity of 95% to afford the formation of 3D cell clusters.

### COMPARATIVE EXAMPLE 1: Cell Culturing

Cells were cultured in the same manner as in Example 2, with the exception that the plates were not coated with REP.

### TEST EXAMPLE 1: Toxicity Test

### Example 1-1: PFOA Toxicity Test

To the cell clusters obtained in Example 2 or the cells obtained in Comparative Example 1 was added a solution of PFOA (perfluorooctanoic acid, Mw 414.1 g/mol, Sigma-Aldrich, 171468-5G, 96%) with a concentration of 5 µM, 20 µM, 50 µM, 100 µM, 200 µM, 300 µM, 400 µM, 500 µM, 600 µM, 700 µM, 800 µM, 900 µM, 1,000 µM or 2,000 µM. After 3, 24 or 48 hrs of incubation, the cells were assayed for proliferation using a cell counting kit-8 (CCK-8, Dojindo, Japan).

The PFOA solution was made by dissolving PFOA in dimethyl sulfoxide (DMSO, Sigma-Aldrich, USA) at a final concentration of 5 µM, 20 µM, 50 µM, 100 µM, 200 µM, 300 µM, 400 µM, 500 µM, 600 µM, 700 µM, 800 µM, 900 µM, 1,000 µM or 2,000 µM.

For the cell proliferation assay, absorbance (OD) at 450 nm was measured on an amicroplate reader (Multiskan, Thermo USA). From the measurements, inhibitory concentration (IC₅₀) values were calculated using TOXCALC 5.0 (Tidepool Scientific Software, USA).

IC₅₀ values determined after incubation with PFOA for 3 hrs, 24 hrs or 48 hrs are summarized in Table 1, below.

The IC₅₀ values given in Table 1 are concentrations of PFOA at which the growth of cells is inhibited by half. A low IC₅₀ value means that a low amount of the toxic substance is needed to inhibit the growth of cells whereas a toxic substance with a high IC₅₀ value is required in a large amount to inhibit the growth of cells.

**TABLE 1**

| | IC₅₀(µM) | | |
|---|---|---|---|
| | 3 hrs | 24 hrs | 48 hrs |
| Cell Cluster of Ex. 2 (A) | 632.15 | 495.09 | 423.2 |
| Cultured Cell of C. Ex. 1 (B) | 511.19 | 388.52 | 309.66 |
| A/B | 1.24 | 1.27 | 1.37 |

As can be seen in Table 1, the IC₅₀ value was detected to be 1.4-fold higher for the cells of Comparative Example 1, compared to the cell cluster of Example 2, indicating that the cell cluster of Example 2 was less susceptible to the toxic substance than were the cells of Comparative Example 1. Thus, the cell cluster was identified to be more suitable for use in in-vitro toxicity tests.

### TEST EXAMPLE 1-2: PFOS Toxicity Test

A solution of PFOS (perfluorooctane sulfonates; Mw 538.2, Sigma-Aldrich, 77282-10G, ≥98.0 (T)) with a concentration of 5 µM, 20 µM, 50 µM, 100 µM, 200 µM, 300 µM, 400 µM, 500 µM, 600 µM, 700 µM, 800 µM, 900 µM, 1,000 µM or 2, 000 µM was added to the cell clusters obtained in Example 2 and to the cells obtained in Comparative Example 1. After 3, 24 or 48 hrs of incubation, the cells were assayed for proliferation using a cell counting kit-8 (CCK-8, Dojindo, Japan).

The PFOS solution was made by dissolving PFOS in dimethyl sulfoxide (DMSO, Sigma-Aldrich, USA) at a final concentration of 5 µM, 20 µM, 50 µM, 100 µM, 200 µM, 300 µM, 400 µM, 500 µM, 600 µM, 700 µM, 800 µM, 900 µM, 1,000 µM or 2,000 µM.

For the cell proliferation assay, absorbance (OD) at 450 nm was measured on an amicroplate reader (Multiskan, Thermo USA). From the measurements, inhibitory concentration (IC₅₀) values were calculated using TOXCALC 5.0 (Tidepool Scientific Software, USA).

IC₅₀ values determined after incubation with PFOS for 3 hrs, 24 hrs or 48 hrs are summarized in Table 2, below.

**TABLE 2**

| | IC₅₀ (µM) | | |
|---|---|---|---|
| | 3 hrs | 24 hrs | 48 hrs |
| Cell Cluster of Ex. 2 (A) | 471.17 | 354.7 | 300.00 |
| Cultured Cell of C. Ex. 1 (B) | 195.9 | 218.75 | 219.88 |
| A/B | 2.41 | 1.62 | 1.37 |

As can be seen in Table 2, the IC₅₀ value was detected to be 1.4- to 2.4-fold higher for the cells of Comparative Example 1, compared to the cell cluster of Example 2, indicating that the cell cluster of Example 2 was less susceptible to the toxic substance than was the cells of Comparative Example 1. Thus, the cell cluster was identified to be more suitable for use in in-vitro toxicity tests.

Designed in consideration of cell-to-cell interaction, the in-vitro toxicity test methods of the present invention, as fully understood from the data of Examples, Comparative Example, and Test Examples, provides results nearer to practical in-vivo toxicity results, compared to conventional in-vitro toxicity test methods.

## Claims

1. An in-vitro toxicity test method for a toxic substance, comprising:
(1) coating a cell culture dish with an artificial elastin-like extracellular matrix, wherein the artificial elastin-like extracellular matrix comprises a protein having the amino acid sequence set forth in the following General Formula 1:
[General Formula 1] TGPG[VGRGD(VGVPG)₆]₂₀;
(2) culturing blast cells in the coated culture dish to form a three-dimensional cell cluster; and
(3) measuring a toxic substance for toxicity to the three-dimensional cell cluster
wherein the toxic substance of step (3) comprises at least one selected from the group consisting of: PFOA (perfluorooctanoic acid) and PFOS (perfluorooctane sulfonate).

2. The in-vitro toxicity test method of claim 1, wherein the cell culture dish is made of at least one material selected from the group consisting of: polystyrene, stainless steel, glass and a biopolymer.

3. The in-vitro toxicity test method of claims 1 or 2, wherein the artificial elastin-like extracellular matrix is applied at an average thickness of 0.001 µm to 10 µm to the culture dish in step (1).

4. The in-vitro toxicity test method of claims 1 to 3, wherein the blast cells of step (2) are selected from the group consisting of: neuroblasts, angioblasts, myoblasts, myeloblasts, fibroblasts, osteoblasts, chondroblasts, and a combination thereof.

5. The in-vitro toxicity test method of claims 1 to 4, wherein the neuroblasts include at least one selected from the group consisting of: N2a, SH-SY5Y, U937, Sk-Mel, A549, BEAS-2, HepG2, beta-TC, HIT-T15, HEK 293, adult stem cells, and embryonic stem cells.

6. The in-vitro toxicity test method of claims 1 to 5, wherein the blast cells of step (2) are cultured at 20°C to 45°C for 12 hrs to 240 hrs.

7. The in-vitro toxicity test method of claims 1 to 6, wherein the coated cell culture dish of step (2) contains at least one selected from the group consisting of: Eagle's Minimum Essential Medium, glutamine, sodium pyruvate, sodium bicarbonate, penicillin, and streptomycin.

8. The in-vitro toxicity test method of claims 1 to 7, wherein the toxicity of step (3) is measured using at least one selected from the group consisting of: an inhibitory concentration (IC₅₀) assay, a cell apoptosis assay, an active oxygen assay, a cytokine assay, and a combination thereof, with preference for an inhibitory concentration (IC₅₀) assay.

9. Use of a medium for an in-vitro toxicity test, the medium comprising:
an artificial elastin-like extracellular matrix-coated culture dish for 3D cell clusters, wherein the artificial elastin-like extracellular matrix comprises a protein having the amino acid sequence set forth in the following General Formula 1:
[General Formula 1] TGPG[VGRGD(VGVPG)₆]₂₀;
and
a 3D cell cluster grown in the culture dish.

10. The use of the medium of claim 9, wherein the 3D cell cluster comprises at least one selected from the group consisting of: neuroblasts, angioblasts, myoblasts, myeloblasts, fibroblasts, osteoblasts, chondroblasts, and a combination thereof.

11. The use of the medium of claims 9 or 10, wherein the artificial elastin-like extracellular matrix is applied at an average thickness of 0.001 µm to 10 µm to the culture dish.

## Patentansprüche

1. In-vitro-Toxizitätstestverfahren für eine toxische Substanz, das Folgendes umfasst:
(1) Beschichten eines Zellkulturgefäßes mit einer künstlichen elastinartigen extrazellulären Matrix, wobei die künstliche elastinartige extrazelluläre Matrix ein Protein mit der Aminosäuresequenz gemäß der folgenden allgemeinen Formel 1 umfasst:
[Allgemeine Formel 1] TGPG[VGRGD(VGVPG)₆]₂₀;
(2) Kultivieren von Blastenzellen in dem beschichteten Kulturgefäß, um zu einem dreidimensionalen Zell-Cluster zu gelangen; und
(3) Messen einer toxischen Substanz in Bezug auf Toxizität gegenüber dem dreidimensionalen Zell-Cluster, wobei die toxische Substanz von Schritt (3) mindestens eine ausgewählt aus der Gruppe bestehend aus PFOA (Perfluoroctansäure) und PFOS (Perfluoroctansulfonat) umfasst.

2. In-vitro-Toxizitätstestverfahren nach Anspruch 1, wobei das Zellkulturgefäß aus mindestens einem Material, ausgewählt aus der Gruppe bestehend aus Polystyrol, Edelstahl, Glas und einem Biopolymer, hergestellt ist.

3. In-vitro-Toxizitätstestverfahren nach den Ansprüchen 1 oder 2, wobei die künstliche elastinartige extrazelluläre Matrix in einer durchschnittlichen Dicke von 0,001 µm bis 10 µm auf das Kulturgefäß in Schritt (1) aufgetragen wird.

4. In-vitro-Toxizitätstestverfahren nach den Ansprüchen 1 bis 3, wobei die Blastenzellen von Schritt (2) aus der Gruppe bestehend aus Neuroblasten, Angioblasten, Myoblasten, Myeloblasten, Fibroblasten, Osteoblasten, Chondroblasten und einer Kombination davon ausgewählt sind.

5. In-vitro-Toxizitätstestverfahren nach den Ansprüchen 1 bis 4, wobei die Neuroblasten mindestens eines, ausgewählt aus der Gruppe bestehend aus N2a, SH-SY5Y, U937, Sk-Mel, A549, BEAS-2, HepG2, beta-TC, HIT-T15, HEK 293, adulten Stammzellen und embryonalen Stammzellen, beinhalten.

6. In-vitro-Toxizitätstestverfahren nach den Ansprüchen 1 bis 5, wobei die Blastenzellen von Schritt (2) 12 h bis 240 h bei 20°C bis 45°C kultiviert werden.

7. In-vitro-Toxizitätstestverfahren nach den Ansprüchen 1 bis 6, wobei das beschichtete Zellkulturgefäß von Schritt (2) mindestens eines, ausgewählt aus der Gruppe bestehend aus Eagle's Minimum Essential Medium, Glutamin, Natriumpyruvat, Natriumbicarbonat, Penicillin und Streptomycin, enthält.

8. In-vitro-Toxizitätstestverfahren nach den Ansprüchen 1 bis 7, wobei die Toxizität von Schritt (3) unter Verwendung von mindestens einem, ausgewählt aus der Gruppe bestehend aus einem Hemmkonzentrations(HK₅₀)-Assay, einem Zellapoptose-Assay, einem Aktivsauerstoff-Assay, einem Cytokin-Assay und einer Kombination davon, vorzugsweise unter Verwendung eines Hemmkonzentrations(HK₅₀)-Assay, gemessen wird.

9. Verwendung eines Mittels für einen In-vitro-Toxizitätstest, wobei das Mittel Folgendes umfasst:
ein mit künstlicher elastinartiger extrazellulärer Matrix beschichtetes Kulturgefäß für 3D-Zell-Cluster, wobei die künstliche elastinartige extrazelluläre Matrix ein Protein mit der Aminosäuresequenz gemäß der folgenden allgemeinen Formel 1 umfasst:
[Allgemeine Formel 1] TGPG[VGRGD(VGVPG)₆]₂₀;
und ein in dem Kulturgefäß gezüchtetes 3D-Zell-Cluster.

10. Verwendung des Mittels nach Anspruch 9, wobei das 3D-Zell-Cluster mindestens eines, ausgewählt aus der Gruppe bestehend aus Neuroblasten, Angioblasten, Myoblasten, Myeloblasten, Fibroblasten, Osteoblasten, Chondroblasten und einer Kombination davon, umfasst.

11. Verwendung des Mittels nach den Ansprüchen 9 oder 10, wobei die künstliche elastinartige extrazelluläre Matrix in einer durchschnittlichen Dicke von 0,001 µm bis 10 µm auf das Kulturgefäß aufgetragen wird.

## Revendications

1. Procédé d'essai de toxicité in vitro pour une substance toxique, comprenant :
(1) le revêtement d'une boîte de culture de cellules avec une matrice extracellulaire de type élastine artificielle, la matrice extracellulaire de type élastine artificielle comprenant une protéine ayant la séquence d'acides aminés décrite dans la formule générale 1 suivante :
[Formule générale 1] TGPG [VGRGD (VGVPG) ₆]₂₀ ;
(2) la culture de cellules blastiques dans la boîte de culture revêtue pour former un agrégat cellulaire tridimensionnel ; et
(3) la mesure d'une substance toxique pour la toxicité pour l'agrégat cellulaire tridimensionnel, la substance toxique de l'étape (3) comprenant au moins l'un choisi dans le groupe constitué de : PFOA (acide perfluorooctanoïque) et PFOS (perfluorooctanesulfonate).

2. Procédé d'essai de toxicité in vitro selon la revendication 1, dans lequel la boîte de culture de cellules est constituée d'au moins un matériau choisi dans le groupe constitué de : polystyrène, acier inoxydable, verre et un biopolymère.

3. Procédé d'essai de toxicité in vitro selon les revendications 1 ou 2, dans lequel la matrice extracellulaire de type élastine artificielle est appliquée à une épaisseur moyenne de 0,001 µm à 10 µm sur la boîte de culture dans l'étape (1).

4. Procédé d'essai de toxicité in vitro selon les revendications 1 à 3, dans lequel les cellules blastiques de l'étape (2) sont choisies dans le groupe constitué de : neuroblastes, angioblastes, myoblastes, myéloblastes, fibroblastes, ostéoblastes, chondroblastes, et une combinaison de ceux-ci.

5. Procédé d'essai de toxicité in vitro selon les revendications 1 à 4, dans lequel les neuroblastes comprennent au moins l'un choisi dans le groupe constitué de : N2a, SH-SY5Y, U937, Sk-Mel, A549, BEAS-2, HepG2, bêta-TC, HIT-T15, HEK 293, cellules souches adultes et cellules souches embryonnaires.

6. Procédé d'essai de toxicité in vitro selon les revendications 1 à 5, dans lequel les cellules blastiques de l'étape (2) sont cultivées à 20 °C à 45 °C pendant 12 h à 240 h.

7. Procédé d'essai de toxicité in vitro selon les revendications 1 à 6, dans lequel la boîte de culture revêtue de cellules de l'étape (2) contient au moins l'un choisi dans le groupe constitué des : milieu essentiel minimal d'Eagle, glutamine, pyruvate de sodium, bicarbonate de sodium, pénicilline et streptomycine.

8. Procédé d'essai de toxicité in vitro selon les revendications 1 à 7, dans lequel la toxicité de l'étape (3) est mesurée en utilisant au moins l'un choisi dans le groupe constitué de : un dosage de concentration inhibitrice (CI₅₀), un dosage d'apoptose cellulaire, un dosage d'oxygène actif, un dosage de cytokine, et une combinaison de ceux-ci, de préférence un dosage de concentration inhibitrice (CI₅₀).

9. Utilisation d'un milieu pour un essai de toxicité in vitro, le milieu comprenant :
une boîte de culture revêtue de matrice extracellulaire de type élastine artificielle pour agrégats cellulaires 3D, la matrice extracellulaire de type élastine artificielle comprenant une protéine ayant la séquence d'acides aminés décrite dans la formule générale 1 suivante :
[Formule générale 1] TGPG[VGRGD(VGVPG)₆]₂₀;
et
un agrégat cellulaire 3D cultivé dans la boîte de culture.

10. Utilisation du milieu selon la revendication 9, dans laquelle l'agrégat cellulaire 3D comprend au moins l'un choisi dans le groupe constitué de : neuroblastes, angioblastes, myoblastes, myéloblastes, fibroblastes, ostéoblastes, chondroblastes, et une combinaison de ceux-ci.

11. Utilisation du milieu selon les revendications 9 ou 10, dans laquelle la matrice extracellulaire de type élastine artificielle est appliquée à une épaisseur moyenne de 0,001 µm à 10 µm sur la boîte de culture.
